(19) Europäisches Patentamt
European Patent Office
Office européen des brevets

(11) Veröffentlichungsnummer: **0 054 215**
**B1**

# (12) EUROPÄISCHE PATENTSCHRIFT

(45) Veröffentlichungstag der Patentschrift: **14.11.84**

(51) Int. Cl.³: **C 07 D 473/12,** A 61 K 31/52 // C07F15/00

(21) Anmeldenummer: **81110026.2**

(22) Anmeldetag: **01.12.81**

## (54) (8-(Dialkylaminoalkoxy)-coffein)-Platinkomplexe, Verfahren zu ihrer Herstellung und sie enthaltende Arzneimittel.

(30) Priorität: **11.12.80 DE 3046927**

(43) Veröffentlichungstag der Anmeldung: **23.06.82 Patentblatt 82/25**

(45) Bekanntmachung des Hinweises auf die Patenterteilung: **14.11.84 Patentblatt 84/46**

(84) Benannte Vertragsstaaten: **AT BE CH DE FR GB IT LI LU NL SE**

(56) Entgegenhaltungen:
**US - A - 2 688 618**

**CHEMICAL ABSTRACTS, Band 89, Nr. 5, 31. Juli 1978, Zusammenfassung Nr. 36571p, Seite 32 COLUMBUS OHIO (US)**
**CHEMICAL ABSTRACTS, Band 91, Nr. 7, 13. August 1979, Zusammenfassung Nr. 49394z, Seite 32 COLUMBUS OHIO (US) A.M. RUSANOV et al.: "Mechanism of the cytostatic effect of proksifein"**
**NATURE, International journal of science, Band 222, 26. April 1969, MacMillan Ltd. LONDON (GB) B. ROSENBERG et al.: "Plastinum compounds: a new class of potent antitumour agents", Seiten 385-386**
**Naturwissenschaften 67 (1981), S. 628**

**Die Akte enthält technische Angaben, die nach dem**

(73) Patentinhaber: **Klosa, Josef, Dr., Jänickestrasse 13, D-1000 Berlin 37 (DE)**

(72) Erfinder: **Klosa, Josef, Dr., Jänickestrasse 13, D-1000 Berlin 37 (DE)**

(74) Vertreter: **UEXKÜLL & STOLBERG Patentanwälte, Beselerstrasse 4, D-2000 Hamburg 52 (DE)**

(56) Entgegenhaltungen: (Fortsetzung)
**Eingang der Anmeldung eingereicht wurden und die nicht in dieser Patentschrift enthalten sind.**

EP 0 054 215 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft [8-(Dialkylaminoalkoxy)-coffein]-Platinkomplexverbindungen, ein Verfahren zu ihrer Herstellung sowie sie enthaltende Arzneimittel.

Die cytostatische Wirkung von bestimmten Platinkoordinationsverbindungen ist bekannt [Rosenberg et al., Nature *222*, 385 bis 386 (1969)]. Es wurden seither verschiedene Klassen von Koordinationskomplexen auf ihre Antitumoraktivität untersucht, vgl. M.G. Cleare, Coordinaten Chemistry Reviews *12*, 349 bis 405 (1974 und A.J. Thomson, Nachr. Chem. Tech. Lab. *25*, 20 bis 23 (1977).

Im Rahmen dieser Bemühungen wurde das cis-Dichlordiamminplatin (DDP) als vielversprechendes Antikrebsmittel entwickelt (DD-PS 142 293). Es handelt sich hierbei um eine anorganische Komplexverbindung, die aus einem zentralen Platinatom umgeben von je zwei Chloratomen und zwei Ammoniakgruppen in cis-Position besteht. Der Nachteil dieser Verbindung ist ihre hohe Giftwirkung; sie lässt sich aus diesem Grunde schwer handhaben und nur unter besonderen Schutzmassnahmen therapheutisch anwenden. Auch der in der DE-OS 3 008 661 beschriebene Versuch, den Nachteilen des DDP durch Herstellung von Organophosphatkomplexen zu begegnen, führte nur zu einem geringen Erfolg, da schon der Umgang mit der hochgiftigen Substanz grosse Schwierigkeiten bereitet.

Es ist ferner bekannt, dass bestimmte Coffein-Derivate, nämlich 8-(Dialkylaminoalkoxy)-coffeine eine gewisse cancerostatische Wirksamkeit besitzen, vgl. J. Klosa, J. prakt. Chem. *6*, 8 bis 13 (1958); ibid. *8*, 117 (1962); A.M. Rusanow et al., Vopr.Radiobiol. Deistvija Tsitostatich Prep. *8*, 80 (1977) [C.A. *91*, 49393y (1979)].

In überraschender Weise zeigte sich nun, dass das 4-wertige Platin in Coffeinkomplexverbindungen bei guter Verträglichkeit schon in Dosen von 10 bis 50 mg/kg Körpergewicht bei der Maus eine überzeugende Antitumorwirkung entfaltet.

Mit den erfindungsgemässen [8-(Dialkylaminoalkoxy)-coffein]-Platinkomplexverbindungenn wird ein Mittel insbesondere zur Behandlung von Tumoren zur Verfügung gestellt, das ausgezeichnet haltbar ist und keinerlei Hautreizungen oder Ätzungen verursacht, so dass sich die umfangreichen Vorsichtsmassnahmen erübrigen, wie sie bei der Verwendung von cis-Dichlordiamminplatin und cis-Dichlordiamminplatin enthaltenden Präparaten notwendig sind.

Erfindungsgemäss werden äquimolare Mengen einer alkoholischen Lösung von 8-(Dialkylaminoalkoxy)-coffein der allgemeinen Formel

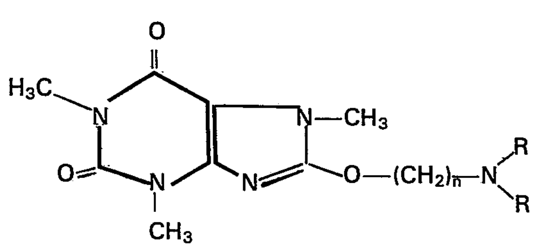

in der n = 2 oder 3 ist und R einen Alkylrest mit 1 bis 3 Kohlenstoffatomen bedeutet, dessen Kette gerade oder verzweigt sein kann, und einer alkoholischen Lösung von Hexachloroplatinsäure miteinander versetzt, wobei neue Komplexverbindungen der allgemeinen Formel

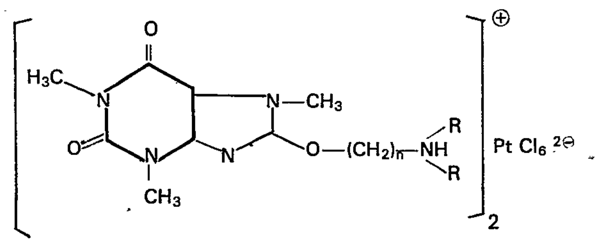

abhängig von ihrer Konstitution entweder sofort oder innerhalb weniger Stunden gebildet werden, die in charakteristisch goldgefärbten Kristallen auskristallisieren und sich gut aus wässrigem Alkohol umkristallisieren lassen. Die neuen Komplexverbindungen können noch Wasser als Kristallwasser binden; sie sind stabil und beliebig lang haltbar.

Die erfindungsgemässen Komplexverbindungen sind stark wirksam gegen experimentell gesetzten Krebs bei Tieren, nämlich Ascites-Tumoren der Maus, während die Ausgangssubstanz Hexachlorplatinsäure unwirksam ist. Die erfindungsgemässen Komplexe stellen durch ihre Ungiftigkeit und daher Ungefährlichkeit im Umgang, ihre Stabilität und ihre unbegrenzte Haltbarkeit einen wesentlichen technischen Fortschritt im Vergleich zu anderen platinhaltigen Krebsmitteln dar.

Die erfindungsgemässen Komplexverbindungen können als Tabletten, Dragees, Lösungen oder Sirups entsprechend den konventionellen Herstellungsmethoden als Arzneimittel, insbesondere als Mittel gegen Krebserkrankungen, zubereitet werden, ohne dass eine Beschränkung der Art und des Bereiches der Anwendung des erfindungsgemässen Arzneimittels vorgesehen ist. Die Erfindung soll nachfolgend anhand einiger Beispiele erläutert werden, ohne auf diese beschränkt zu sein.

*Beispiel 1*

Herstellung von Di-[8-(2-diisopropylaminoethoxy)--coffein]-hexachloroplatinat.

10 ml Diisopropylamino-ethanol wurden in 50 ml Toluol gelöst. In diese Lösung wurden1,2 g Natrium in Scheiben geschnitten eingebracht. Es wurde 6 bis 8 Stunden lang auf 50 bis 60°C erhitzt. In dieser Zeit löst sich alles Natrium unter Bildung des Natriumsalzes des Diisopropylamino-ethanols auf. Alsdann wurden unter Rühren portionsweise 11 g gut getrocknetes 8-Chlorcoffein innerhalb von 20 Minuten eingetragen. Anschliessend wurde 20 Minuten lang auf dem Wasserbad auf 40 bis 50°C erhitzt und dann das Reaktionsgut 5 Stunden lang sich selbst überlassen, vom Kochsalz abgesaugt und das Filtrat auf dem Wasserbad im Vakuum vom Toluol befreit. Der ölige Rückstand wurde mit wenig Methanol angeteigt, so dass derselbe in farblosen Kristallen kristallisierte. Die so gewonnene Substanz zeigte einen Fp. von 72 bis 74°C, sie wurde durch Lösen in Methanol und Zusatz von Wasser umkristallisiert, der Fp. lag dann zwischen 90 und 92°C. Die Ausbeute betrug 12 g.

6,6 g des so erhaltenen 8-(Diisopropylaminoethoxy)-coffeins wurden in 20 ml Methanol gelöst. In die wasserklare Lösung wurde eine Lösung von 5,2 g Hexachloroplatin(IV)-säure als Hexahydrat in 10 ml Methanol eingetropft. Es fielen sofort goldgelbgefärbte Kristalle aus. Zur Vervollständigung der Kristallisation wurde noch mit 20 ml Aceton verdünnt und der Ansatz wurde 2 Stunden lang stehengelassen, abgesaugt, mit wenig Aceton gewaschen und auf Ton getrocknet. Die Fp.-Bestimmung ergab ab 190°C Braunfärbung, bei 218 bis 220°C unter Zersetzung eine teerartig schwarze Schmelze. Nach Umkristallisation durch Lösen in 60%igem heissen Alkohol und Erkaltenlassen wurden goldgelbe balkenförmige Kristalle erhalten.

*Analyse:*

Fp. ab 190°C Braunfärbung, 220°C unter Zersetzung teerartige schwarze Schmelze, Ausbeute: 9,5 g.

$(C_{16}H_{28}N_5O_3)_2PtCl_6$
Mol-Gew. 1028,35
Ber.: Pt 18,02%
Gef.: Pt 18,10%.

*Beispiel 2*

Herstellung von Di-[8-(3-dimethylamino-propoxy)-coffein]-hexachloroplatinat.

6,6 g 8-(3-Dimethylaminopropoxy)-coffein, dargestellt nach J. Klosa [J. prakt. Chemie, *6*, 8 bis 13 (1958)], vom Fp. 58 bis 60°C wurden in 30 ml Methanol gelöst. In diese Lösung wurden unter Rühren bei Raumtemperatur innerhalb von 10 Minuten 5,2 g Hexachloroplatin(IV)-säure als Hexahydrat in 20 ml Methanol eingetropft. Es fiel ein goldgelber sandiger Niederschlag aus, der nach 6 Stunden Stehen abgesaugt und mit Aceton gewaschen wurde.

*Analyse*

Fp. 180 bis 182°C, Schmelze war orange gefärbt. Ausbeute: 10 g. Das Produkt enthielt 3 Mol Wasser als Hydratwasser.

$(C_{13}H_{22}N_5O_3)_2PtCl_6 \cdot 3H_2O$
Ber.: Pt 18,54%
Gef.: Pt 18,35%.

Der neue Komplex ist wasserlöslich.

Nach dem gleichen Verfahren wurde aus 8-(2-Diethylamino-ethoxy)-coffein und Hexachloroplatin(IV)-säure Di-[8-(2-diethylamino-ethoxy)-coffein]-hexachloroplatinat hergestellt; der Fp. lag zwischen 194 und 196°C, die Ausbeute betrug ca. 80%.

*Beispiel 3*

Herstellung pharmazeutischer Formulierungen.

a) *Kapseln*

Die folgenden Bestandteile wurden gemischt und in Kapseln zu 100 mg gefüllt:

| | |
|---|---|
| Di-[8-(3-dimethylaminopropoxy)-coffein-]-hexachloroplatinat nach Beispiel 2 | 30 mg |
| Milchzucker | 70 mg |

b) *Tabletten*

Die folgenden Substanzen wurden gemischt, sodann auf einem Kompaktor agglomeriert, daraufhin granuliert und zu Tabletten oder Dragees gerpesst:

| | |
|---|---|
| Di-[8-(3-dimethylaminopropoxy)-coffein]-hexachloroplatinat nach Beispiel 2 | 50 mg |
| Hydroxyethylcellulose | 30 mg |
| Polyvinylpyrrolidin | 10 mg |
| Talkum | 6 mg |
| Magnesiumstearat | 4 mg |
| | 100 |

c) *Ampullen*

| | |
|---|---|
| Komponenten: Di-[8-(3-dimethylamino-propoxy)-coffein]-hexachloroplatinat nach Beispiel 2 | 60 g |
| Natriumchlorid | 16 g |
| bidestilliertes Wasser ad | 2000 g |

Das Coffeinplatinat nach Beispiel 2 wurde mit dem Natriumchlorid in dem frisch destillierten Wasser gelöst. Die Lösung wurde filtriert und in 2-ml-Ampullen geüllt. Nach dem Zuschmelzen wurden die Ampullen im Dampfsterilisator bei 120°C 30 Minuten lang sterilisiert.

*Beispiel 4*

Untersuchung der carcinostatischen Wirkung von Di-[8-(3-dimethylaminopropoxy)-coffein]-hexachloro-platinat an Mäusen und Ratten.

Zur Behandlung von Ehrlich-Tumoren im Bereich der Pfote von Mäusen wurden dreimal im Abstand von jeweils zwei Tagen 50 mg Di-[8-(3-dimethylaminopropoxy)-coffein]-hexachloroplatinat je kg Körpergewicht intravenös injiziert. In 70% der Fälle trat eine Heilung innerhalb weniger Tage ein.

In entsprechenden Untersuchungsreihen mit gleicher Dosis wie oben angegeben konnte das Wachstum von Ehrlich-Ascites-Zellen immer gehemmt und in 15% der Fälle Heilung erzielt werden.

In ergänzenden Untersuchungen am Sarkom 180 der Maus und am Yoshida-Sarkom der Ratte (beide in Ascites-Form) wurde in allen Fällen eine Wachstumshemmung beobachtet.

**Patentansprüche**

1. [8-(Dialkylaminoalkoxy)-coffein]-hexachloroplatinate der allgemeinen Formel

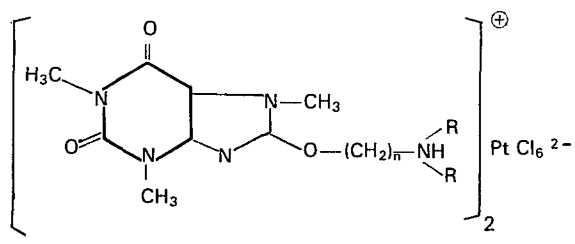

in der n = 2 oder 3 ist und R einen Alkylrest mit 1 bis 3 Kohlenstoffatomen bedeutet, dessen Kette gerade oder verzweigt sein kann.

2. Verfahren zur Herstellung der [8-(Dialkylaminoalkoxy)-coffein]-hexachloroplatinate nach Anspruch 1, dadurch gekennzeichnet, dass ein 8-(Dialkylaminoalkoxy)-coffein der allgemeinen Formel

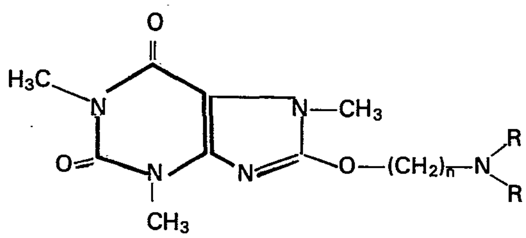

in der n und R die obige Bedeutung haben, mit Hexachloroplatinsäure $H_2PtCl_6$ bei Raumtemperatur und schonenden Bedingungen umgesetzt wird.

3. Verfahren nach Anspruch 2, dadurch gekennzeichnet, dass die Hexachloroplatinsäure in Hydratform eingesetzt wird.

4. Pharmazeutisches Mittel, dadurch gekennzeichnet, dass es als Wirkstoff eine Verbindung nach Anspruch 1 in Kombination mit einem pharmazeutisch verträglichen Träger enthält.

5. Verbindung nach Anspruch 1 zur Verwendung als Cytostatikum in Arzneimitteln der Human- und Veterinärmedizin.

## Claims

1. [8-(Dialkylamino alkoxy)caffeine]hexachloroplatinates of the general formula

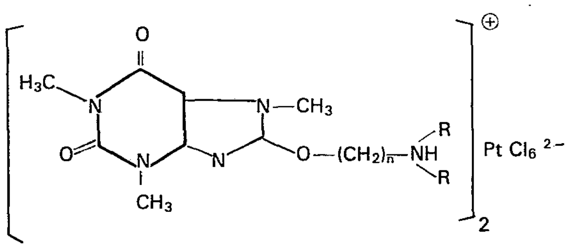

wherein n is 2 or 3 and R is an alkyl group with 1 to 3 carbon atoms, the chain of carbon atoms being straight or branched.

2. A process of preparing the [8-(dialkylamino alkoxy)-caffeine]hexachloroplatinates according to claim 1, characterized in that an 8-(dialkylamino alkoxy)-caffeine of the general formula

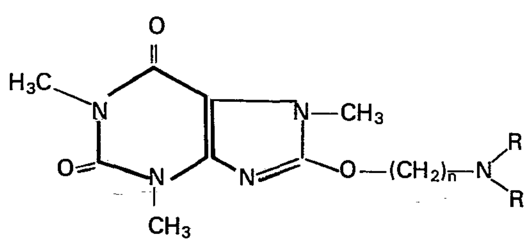

wherein n is 2 or 3 and R is an alkyl group with 1 to 3 carbon atoms, the chhain of carbon atoms being straight or branched is caused to react with hexachloro platinic acid $H_2PtCl_6$ at room temperature and under mild conditions.

3. The process according to claim 2, characterized in that the hexachloro platinic acid is used in the hydrate form.

4. Pharmaceutical product, characterized in that it contains as an active component a compound of claim 1 in combination with a pharmaceutically acceptable carrier.

5. Compound according to claim 1 for use in cytostatic remedy in pharmaceutical products for application in human and veterinary medicine.

## Revendications

1. Hexachloroplatinate de [8-(dialkylaminoalkoxy)-caféine] de formule générale:

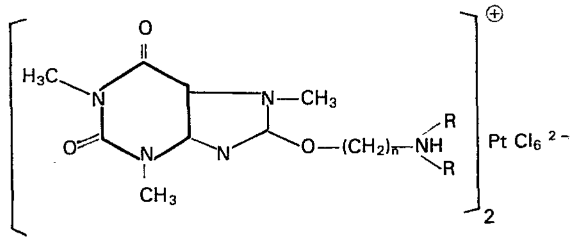

dans laquelle
n = 2 ou 3; et
R représente un radical alkyle possédant 1 à 3 atomes de carbone et dont la chaîne peut être droite ou ramifiée.

2. Procédé de préparation de hexachloroplatinate de [8-(dialkylaminoalkoxy)-caféine] selon la revendication 1, caractérisé en ce que l'on fait réagir une 8-(dialkylaminoalkoxy)-caféine de formule générale:

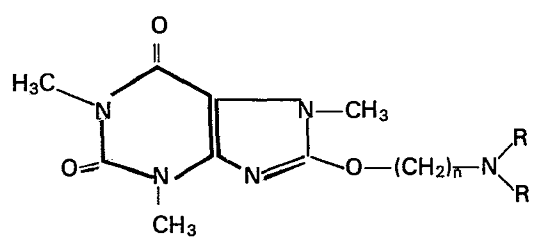

dans laquelle
n et R ont les significations ci-dessus
avec de l'acide hexachloroplatinique $H_2PtCl_6$ à la température ambiante et dans des conditions ménagées.

3. Procédé selon la revendication 1, caractérisé en ce que l'on utilise l'acide hexachloroplatinique sous la forme de son hydrate.

4. Produit pharmaceutique caractérisé en ce qu'il contient comme substance un composé selon la revendication 1, en combinaison avec un vecteur pharmaceutique compatible.

5. Composé selon la revendication 1, pour utilisation comme cytostatique dans des médicaments de la médicine humaine et vétérinaire.